# EUROPEAN PATENT SPECIFICATION

(11) **EP 0 606 333 B1**
(45) Date of publication and mention of the grant of the patent: **12.03.1997**
(21) Application number: 92920695.1
(22) Date of filing: 30.09.1992
(51) Int. Cl.: C10G 25/00, C07C 7/12, B01J 20/06

(54) **DESULPHURISATION TREATMENT**
ENTSCHWEFELUNGSBEHANDLUNG
TRAITEMENT DE DESULFURATION

(30) Priority: 03.10.1991 GB 9120973
(43) Date of publication of application: 20.07.1994
(73) Proprietor: DYTECH CORPORATION LIMITED, Stannington, Sheffield S6 6BW (GB)
(72) Inventor: HOLT, Andrew Dytech Corporation, Stannington Sheffield S6 6BW (GB)
(74) Representative: Shaw, Laurence
(86) International application number: PCT/GB92/01799
(87) International publication number: WO 93/07237

(56) References cited:
- EP-A- 0 014 579
- EP-A- 0 207 645
- EP-A- 0 429 053
- EP-A- 0 469 513
- GB-A- 2 202 546
- US-A- 4 888 157

## Description

The invention relates to desulphurisation treatment and in particular to the desulphurisation treatment of hydrocarbon streams (liquid or gas or both) by the partial or complete removal of sulphur compounds, e.g. hydrogen sulphide, low molecular weight mercaptans, or the like therefrom.

EP-A-0429053 discloses a method of removing trialkyl arsines from a fluid, particularly a gas, in which the fluid is contacted with a solid sorbent material containing a manganese oxide, preferably MnO2, and at least one Group 1B metal oxide, preferably CuO. The treatment temperature may be in the range - 20° to 100°C.

US-A-4888157 discloses a method of desulphurising carbon dioxide - containing gas streams using agglommerates containing copper oxide, zinc oxide and/or an oxide of another element, manganese, aluminium, silicon, titanium, zirconium and chromium being specified, with aluminium oxide being preferred. Treatment temperatures are below 100°C.

The present invention aims to provide an improved means of desulphurisation of hydrocarbon streams.

In one aspect the invention provides a method of reducing the content of a sulphur compound in a hydrocarbon stream, the method comprising reacting the stream with a derivative of manganese and a promoter **characterised in that** the promoter is one or more of potassium hydroxide, nickel hydroxide and sodium hydroxide and that the reaction is carried out at a temperature from about ambient up to about 150°C.

Preferably the temperature is substantially ambient.

Preferably the manganese derivate is the oxide, hydroxide and/or carbonate, or the like. The invention is based on the novel realisation that manganese dioxide or other derivative with a suitable promoter is an efficient agent for the removal of sulphur compounds at low temperature, i.e. up to about 150°C. The agent may be used neat or it may be included in other agents, e.g. mixtures based on promoted calcium derivatives, in major or minor proportions.

In a preferred feature, a zinc derivative, such as the oxide, hydroxide and/or carbonate, is present.

The sulphur compound to be removed may be hydrogen sulphide gas or a low molecular weight mercaptan such as propyl mercaptan. The hydrocarbon stream may be liquid or gas or both, examples being natural gas, town gas, industrial waste gas, coke oven gas, coal gas, liquid or gas from a petroleum plant or oil refinery.

In yet another aspect the invention provides for use in the method a composition of a derivative of manganese and a promoter, the composition being in the form of bonded granules and the promoter being one or more of potassium hydroxide, nickel hydroxide and sodium hydroxide. Optionally a derivative of zinc may be included. The bonding agent may be a cement, alumina or a clay, silica, organic resin; or the like.

It is believed that the use of the specified promoter in association with a manganese derivative is advantageous even when used at higher temperature, i.e. above 150°C.

The temperatures may be any of those used conventionally and as are well known to the skilled man of the art.

In order that the invention may be well understood it will now be described by way of illustration with reference to the following examples.

### Example I

Zinc oxide powder (50g) and manganese dioxide (50g) and calcium aluminate cement (5g) were dry mixed for 15 minutes. Water was added to the mix in sufficient quantity to form granules which were shaped to be of 3 mm mean diameter. These were dried at 150°C for 5 hours.

### Example II

The method of Example I was repeated adding nickel hydroxide powder (3%) to the dry mix and potassium hydroxide (5%) to the water.

### Example III

The ability of different materials specified in the Table was tested by keeping them in a closed vessel containing hydrogen sulphide gas at 15°C for 24 hours. The manganese dioxide was obtained from two sources, identified as (a) and (b). The uptake of the gas was determined by the increase in weight. The results are also shown in the Table from which it is clear that manganese dioxide with the promoter of the invention is an efficient agent for removing the gas.

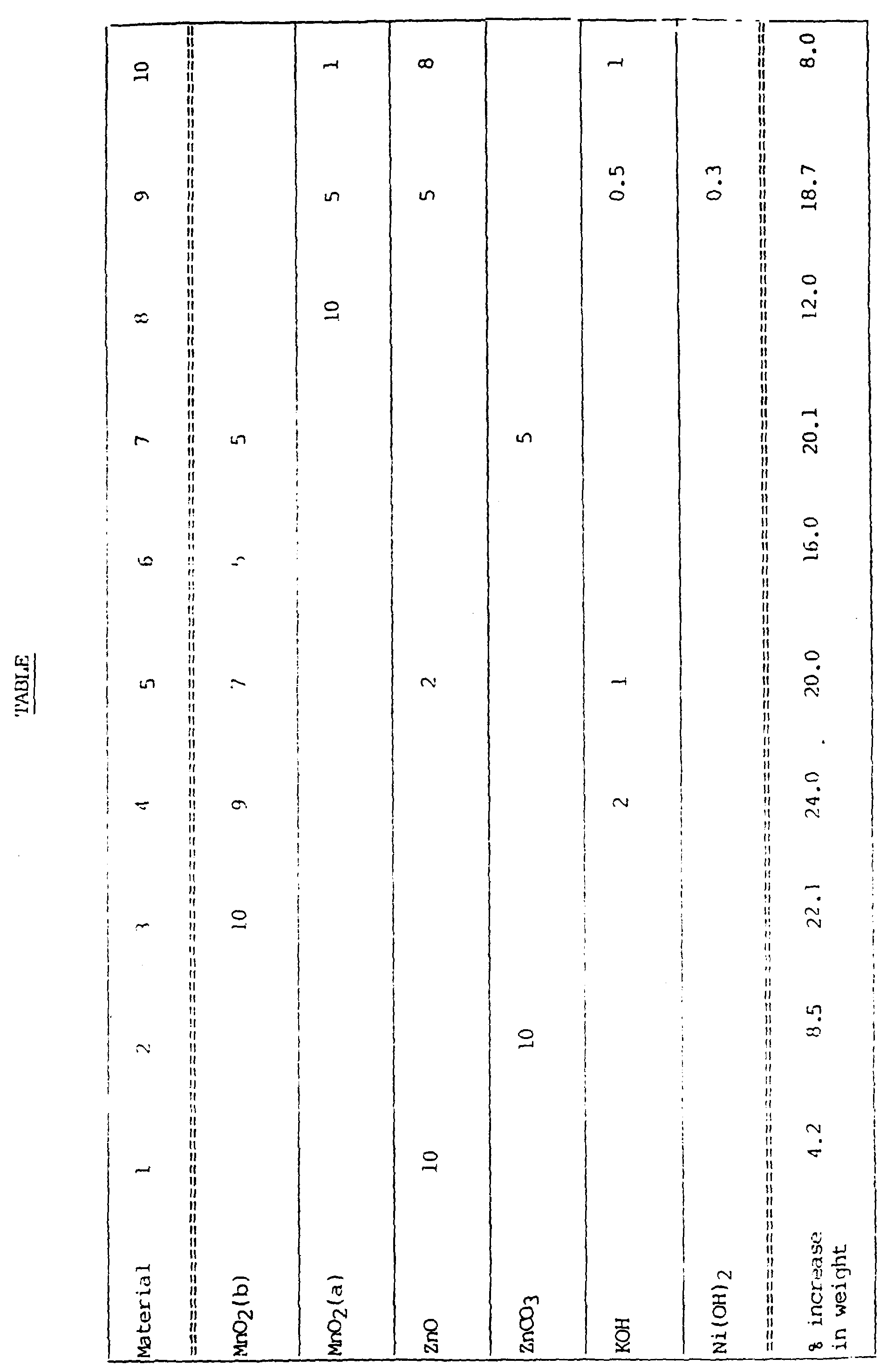

## Claims

1. A method of reducing the content of a sulphur compound in a hydrocarbon stream, the method comprising reacting the stream with a derivative of manganese and a promoter **characterised in that** the promoter is one or more of potassium hydroxide, nickel hydroxide and sodium hydroxide and that the reaction is carried out at a temperature from ambient up to 150°C.

2. A method according to Claim 1, wherein the reaction temperature is substantially ambient.

3. A method according to Claim 1 or 2, wherein the manganese derivative is the oxide, dioxide, hydroxide and/or carbonate.

4. A method according to Claim 1, 2 or 3, wherein a zinc derivative is also included.

5. A method according to Claim 4, wherein the zinc derivation is the oxide, hydroxide and/or carbonate.

6. A method according to any preceding Claim, wherein the sulphur compound to be removed is hydrogen sulphide gas or a low molecular weight mercaptan from a hydrocarbon stream which is natural gas, town gas, industrial waste gas, coke oven gas, coal gas, liquid or gas from a petroleum plant or oil refinery.

7. A composition for use in a method according to any preceding Claim, the composition comprising a derivative of manganese and a promoter, the composition being in the form of bonded granules and the promoter being one or more of potassium hydroxide, nickel hydroxide and sodium hydroxide.

8. A composition according to Claim 7, wherein the bonding agent is cement, alumina or a clay, silica, and organic resin.

9. A composition according to Claim 7 or 8, including a zinc derivative.

## Patentansprüche

1. Verfahren zur Reduzierung des Gehalts einer Schwefelverbindung in einem Kohlenwasserstoffstrom, umfassend das Reagieren des Stroms mit einem Manganderivat und einem Promotor, dadurch gekennzeichnet, daß als Promotor ein oder mehrere Elemente aus der Gruppe bestehend aus Kaliumperoxid, Nickelhydroxid und Natriumhydroxid gewählt wird/werden und daß die Reaktion bei einer Temperatur zwischen Umgebungstemperatur und 150°C durchgeführt wird.

2. Verfahren nach Anspruch 1, bei dem die Reaktionstemperatur im wesentlichen Umgebungstemperatur ist.

3. Verfahren nach Anspruch 1 oder 2, bei dem das Manganderivat das Oxid, das Dioxid, das Hydroxid und/oder das Karbonat ist.

4. Verfahren nach Anspruch 1, 2 oder 3, bei dem auch ein Zinkderivat enthalten ist.

5. Verfahren nach Anspruch 4, bei dem das Zinkderivat das Oxid, das Hydroxid und/oder das Karbonat ist.

6. Verfahren nach einem der vorhergehenden Ansprüche, bei dem die zu entfernende Schwefelverbindung Schwefelwasserstoffgas oder ein Mercaptan mit niedrigem Molokülargewicht von einem Kohlenwasserstoffstrom ist, der Erdgas, Stadtgas, Industrieabgas, Koksofengas, Kohlegas, Flüssigkeit oder Gas aus einer Petrolanlage oder einer Ölraffinerie ist.

7. Komposition zur Verwendung in einem Verfahren nach einem der vorhergehenden Ansprüche, wobei die Komposition ein Manganderivat und einen Promotor umfaßt, wobei die Komposition die Form eines gebundenen Granulats hat und der Promotor ein oder mehrere Elemente aus der Gruppe bestehend aus Kaliumhydroxid, Nickelhydroxid und Natriumhydroxid ist.

8. Komposition nach Anspruch 7, bei der das Bindemittel Zement, Aluminiumoxid oder ein Ton, Silika oder organischer Harz ist.

9. Komposition nach Anspruch 7 oder 8, umfassend ein Zinkderivat.

## Revendications

1. Méthode visant à réduire la teneur en composé sulfurique d'un flux d'hydrocarbure, la méthode comprenant la réaction du flux avec un dérivé de manganèse et un promoteur, caractérisée en ce que le promoteur consiste en l'un ou plusieurs des hydroxydes suivants : de potassium, de nickel et de sodium et en ce que la réaction est réalisée sous une température comprise entre la température ambiante et 150°C.

2. Méthode, selon la revendication 1, dans laquelle la température de la réaction est essentiellement ambiante.

3. Méthode, selon les revendications 1 ou 2, dans laquelle le dérivé du manganèse est de l'oxyde, du dioxyde, de l'hydroxyde et/ou du carbonate.

4. Méthode, selon les revendications 1, 2 ou 3, dans laquelle un dérivé du zinc est également inclus.

5. Méthode, selon la revendication 4, dans laquelle le dérivé du zinc est de l'oxyde, de l'hydroxyde et/ou du carbonate.

6. Méthode, selon toute revendication précédente, dans laquelle le composé sulfurique qui doit être extrait est un gaz d'acide sulfhydrique ou un mercaptan à faible poids moléculaire provenant d'un flux d'hydrocarbure consistant en gaz naturel, gaz de ville, gaz provenant de déchets industriels, gaz de four à coke, gaz de charbon, d'un liquide ou d'un gaz provenant d'une usine ou d'une raffinerie de pétrole.

7. Composition qui doit être utilisée dans une méthode conformément à toute revendication précédente, la composition comprenant un dérivé de manganèse et un promoteur, la composition se présentant sous forme de granules liés et le promoteur, sous forme d'un ou plusieurs des hydroxydes suivants : de potassium, de nickel et de sodium.

8. Composition, conformément à la revendication 7, dans laquelle l'agent de liaison est du ciment, de l'aluminium ou de l'argile, du silice et une résine organique.

9. Composition, selon les revendications 7 ou 8, comprenant un dérivé du zinc.
